# EUROPÄISCHE PATENTSCHRIFT

(11) **EP 0 701 999 B1**
(45) Veröffentlichungstag und Bekanntmachung des Hinweises auf die Patenterteilung: **19.08.1998**
(21) Anmeldenummer: 95113650.6
(22) Anmeldetag: 31.08.1995
(51) Int. Cl.: C07C 233/36, C07C 229/12

(54) **Verfahren zur Herstellung von Betainen**
Process for the preparation of betaines
Procédé de préparation de bétaines

(30) Priorität: 16.09.1994 DE 4433062
(43) Veröffentlichungstag der Anmeldung: 20.03.1996
(73) Patentinhaber: Th. Goldschmidt AG, 45127 Essen (DE)
(72) Erfinder: Foitzik, Willi, D-46238 Bottrop (DE); Grüning, Burghard, Dr., D-45134 Essen (DE); Käseborn, Hans-Dieter, D-45134 Essen (DE); Passmann, Michael, D-45481 Mülheim (DE); Weitemeyer, Christian, Dr., D-45134 Essen (DE)

(56) Entgegenhaltungen:
- EP-A- 0 557 835
- WO-A-91/08193
- DE-C- 4 309 900

## Beschreibung

Die Erfindung betrifft ein Verfahren zur Herstellung von Betainen der allgemeinen Formel 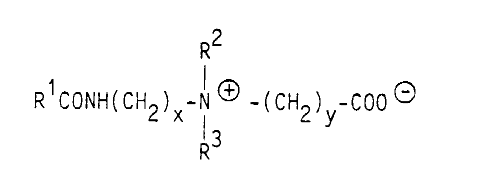 wobei
- R¹CO: ein von einer Fettsäure oder einem Fettsäuregemisch mit 6 bis 18 Kohlenstoffatomen abgeleiteter Acylrest ist,
- R² und R³: gleich oder verschieden sind und Alkylreste mit 1 bis 4 Kohlenstoffatomen bedeuten,
- x =: 2 oder 3 und
- y =: 1, 2 oder 3 ist,
durch Quaternierung der Fettsäureamiddialkylamine der allgemeinen Formel 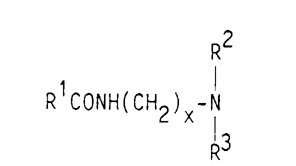 mit ω-Halogenalkylcarbonsäuren der Formel X-(CH₂)_{y}COOH, wobei X ein Halogenrest ist, oder deren Salzen in wäßriger Lösung.

Die Erfindung betrifft insbesondere ein Verfahren, welches die Herstellung von Betainen ermöglicht, die frei von organisch gebundenem Chlor und insbesondere frei von Natriummono- und -dichloracetat sind.

Als Carbonsäuren R¹COOH können insbesondere gesättigte und ungesättigte Fettsäuren eingesetzt werden, wobei auch Gemische von Fettsäuren, insbesondere Cocosfettsäure und Palmkernfettsäure, verwendet werden können.

Betaine werden in großem Umfang zur Herstellung von Körperreinigungsmitteln, insbesondere zur Herstellung von Haarwaschmitteln verwendet. Man ist deshalb bestrebt, die Betaine frei von Verunreinigungen herzustellen, welche Hautreizungen verursachen können oder in sonstiger Weise aus physiologischen Gründen unerwünscht sind.

In der DE-PS 29 26 479 ist ein Verfahren der obenbezeichneten Gattung beschrieben, welches dadurch gekennzeichnet ist, daß man die Quaternierungsreaktion während des gesamten Reaktionsablaufes in alkalischer Lösung, die bei 98 °C gemessen einen pH-Wert von 7,5 bis 10,5 aufweist, durchführt.

Durch die Einhaltung des pH-Wertes bei der Quaternierungsreaktion wird erreicht, daß sich nach einer Reaktionszeit von etwa drei Stunden dünnschichtchromatographisch kein Fettsäureamiddialkylamin mehr nachweisen läßt, wobei die Nachweisgrenze bei etwa 0,02 Gew.-% liegt. Verlängert man die Reaktionszeit auf etwa 8 bis 10 Stunden, verringert sich auch der Gehalt an organisch gebundenem Chlor im Umsetzungsprodukt. Mit Verfeinerung der Analysenmethoden hat sich jedoch gezeigt, daß auch unter diesen Bedingungen im erhaltenen Betain noch Restmengen an Verbindungen mit organisch gebundenem Chlor, insbesondere Natriumdichloracetat enthalten sind. Die Anwendung höherer pH-Werte als 10,5 führt auch nur in unzureichendem Maße zu einer Verringerung der Restbestandteile an Chloressigsäuren. Hierzu wären übermäßig lange Reaktionszeiten notwendig, die sich aus wirtschaftlichen Gründen verbieten. Darüber hinaus besteht die Gefahr einer zunehmenden Zersetzung der Ausgangsprodukte bzw. der entstehenden Betaine, u.a. verursacht durch Hydrolyse der Amidbindung. Oberhalb eines pH-Wertes von 10,5 ist auch mit einer Zersetzung des Produktes zu rechnen.

Die DE-OS 39 39 264 betrifft ein Verfahren zur Erniedrigung des Restgehaltes an freiem Alkylierungsmittel in wäßrigen Lösungen amphoterer oder zwitterionischer Tenside, mit dem Kennzeichen, daß man die Lösungen mit Ammoniak, einer Aminosäure mit 2 bis 8 Kohlenstoffatomen oder einem Oligopeptid nachbehandelt. Durch diese Nachbehandlung soll der Restgehalt an freiem Alkylierungsmittel, insbesondere an Chloressigsäure, auf Werte unterhalb von 0,01 Gew.-% (bezogen auf Feststoffgehalt) gesenkt werden. Ein wesentlicher Nachteil dieses Verfahrens besteht jedoch darin, daß ein zusätzlicher Verfahrensschritt benötigt wird. Ein weiterer Nachteil ist darin zu erkennen, daß die Umsetzungsprodukte des Alkylierungsmittels mit Ammoniak, einer Aminosäure oder einem Oligopeptid im Verfahrensprodukt als Verunreinigung verbleiben.

Die DE-OS 42 32 157 betrifft ein Verfahren zur Herstellung von reinen wäßrigen Betainlösungen, wobei die Betainlösung zur Umwandlung noch vorhandener Chloressigsäuren mit einem Sulfonierungsagens behandelt wird.

Auch hier ist also eine Fremdstoffzugabe mit zusätzlichen Reinigungsschritten erforderlich.

In der DE-OS 42 05 880 wird ein Verfahren zur Herstellung von Betainen durch Quaternierung von Fettsäureamiddialkylaminen mit ω-Halogenalkylcarbonsäuren der Formel X-(CH₂)_{y}COOH beschrieben, wobei X ein Halogenrest ist, oder deren Salzen in wäßriger Lösung, wobei man die Reaktion innerhalb eines Temperaturbereiches von 115 bis 180 °C durchführt, bis kein organisch gebundenes Chlor mehr nachweisbar ist, wobei man gegebenenfalls in einer ersten Stufe bei 80 bis 100 °C das Fettsäureamiddialkylamin partiell oder vollständig quaterniert.

Dabei ist die Untergrenze des Temperaturbereiches von 115 °C durch den Eintritt des Abbaus von Natriumdichloracetat gegeben. Unterhalb dieser Temperatur verläuft der Abbau nicht oder innerhalb eines Zeitraumes, der für ein wirtschaftlich durchzuführendes Verfahren nicht annehmbar ist. Als Obergrenze des Temperaturbereiches wird 180 °C wegen der dann beginnenden Zersetzung des Verfahrensproduktes bzw. der Reaktionspartner angegeben.

Die vorliegende Erfindung befaßt sich ebenfalls mit dem technischen Problem der Herstellung eines Betains, welches frei von Verunreinigungen ist, insbesondere frei von Verbindungen mit organisch gebundenem Chlor, wie Natriummono- und -dichloracetat, und ohne Fremdstoffzugabe durchführbar ist.

Erfindungsgemäß gelingt dies überraschenderweise dadurch, daß man in einer ersten Stufe bei 80 bis 100 °C das Fettsäureamiddialkylamin partiell oder vollständig quaterniert und die Reaktion in einer zweiten Stufe oberhalb eine Temperatur von 180 °C durchführt, bis kein organisch gebundenes Chlor mehr nachweisbar ist.

Vorzugsweise führt man die Reaktion bei einer Temperatur von 185 bis 250 °C, insbesondere bei einer Temperatur von 185 bis 240 °C durch. Die Reaktionsdauer, nach der kein organisch gebundenes Chlor mehr nachweisbar ist, beträgt in Abhängigkeit von der Temperatur 1 bis 25 Minuten.

Die erforderliche Reaktionsdauer ist u.a. abhängig von der Temperatur. Bei einer relativ niedrigen Temperatur von z.B. 185 °C wird man eine ausreichend lange Reaktionszeit von mindestens 15 Minuten wählen; andererseits wird man bei einer höheren Temperatur von z.B. 240 °C eine kurze Reaktionszeit von z.B. 1 Minute wählen, um einerseits kein organisch gebundenes Chlor mehr im Produkt nachweisbar zu haben und andererseits eine Zersetzung des Betains zu vermeiden.

Das Verfahren wird bevorzugt bei pH-Werten im Bereich von 6 bis 10 durchgeführt. Es kann aber auch bei extremen pH-Werten angewendet werden, wobei die Reaktionsbedingungen, wie Temperatur und Verweilzeit, gegebenenfalls anzupassen sind.

Da bei der erhöhten Temperatur die Quaternierungsreaktion und die Abbaureaktion des organisch gebundenen Chlors konkurrierend verlaufen, müßte bei der einstufigen Reaktionsweise eine überstöchiometrische Menge Halogenalkylcarbonsäure eingesetzt werden. Führt man jedoch die Quaternierungsreaktion bereits in einer ersten Stufe bei einer Temperatur von 80 bis 100 °C partiell oder vollständig durch, ist es möglich, die Umsetzung mit stöchiometrischen Mengen oder mit einem nur geringen überschuß an Halogenalkylcarbonsäure durchzuführen.

Es ist im Hinblick auf die gewählten Temperaturen erforderlich, in einem geschlossenen System, wie z.B. in einem entsprechend dimensionierten Rührautoklaven zu arbeiten. Besonders vorteilhaft ist die Verwendung eines Rohrreaktors, bei dem die Reaktionsdauer, insbesondere die der zweiten Reaktionsstufe, durch entsprechende Einstellung der Verweilzeiten geregelt werden kann.

Mit dem erfindungsgemäßen Verfahren gelingt es, den Gehalt der Betainlösung an Natriummonochloracetat und Natriumdichloracetat unter die jeweilige Nachweisgrenze von < 10 ppm zu senken.

Dies ist überraschend angesichts der Tatsache, daß oberhalb von 180 °C die Zersetzung des Verfahrensproduktes bzw. der Reaktionspartner beginnt.

Das erfindungsgemäße Verfahren hat den Vorteil, daß dem Produkt zum Abbau der organischen Chlorverbindungen keine das Produkt verunreinigenden Reagenzien zugesetzt werden müssen. Es erlaubt zudem gegenüber dem Stand der Technik erheblich verkürzte Reaktionszeiten.

In den folgenden Beispielen wird das erfindungsgemäße Verfahren im Vergleich zu den Verfahren des Standes der Technik näher erläutert.

### Beispiele

### Herstellung einer wäßrigen Lösung von Cocosfettsäureamidopropylbetain

Das als Ausgangsprodukt verwendete Cocosfettsäureaminoamid wird durch Amidierung von Cocosfettsäure mit 3-N,N-Dimethylaminopropylamin hergestellt. Es weist einen durch Titration ermittelten Gehalt an tertiärem Stickstoff von 4,6 Gew.-% N auf. Die Titration erfolgt mit 0,1 M Salzsäure. Als Indikator wird Bromphenolblau verwendet. Als Natriummonochloracetat wird ein Produkt handelsüblicher Qualität eingesetzt, welches etwa 0,1 Gew.-% Natriumdichloracetat als Verunreinigung enthält.

3600 g Cocosfettsäureaminoamid werden zu einer Lösung von 1280 g (11 Mol) Natriumchloracetat in 8280 g Wasser gegeben, mit 53 g 40 %iger wäßriger Natronlauge versetzt und im Dreihalskolben mit Rührer auf 98 °C erhitzt. In regelmäßigen Abständen werden Proben genommen und hinsichtlich ihres Gehaltes an Natriummonochloracetat und Natriumdichloracetat mittels Kapillarelektrophorese untersucht.

Der Gehalt an Natriummonochloracetat beträgt nach 2 Stunden Reaktionszeit 1200 ppm und nach 8 Stunden Reaktionszeit < 10 ppm, der Gehalt an Natriumdichloracetat beträgt nach 2 Stunden Reaktionszeit 52 ppm und nach 8 Stunden Reaktionszeit 35 ppm.

Ein Teil des Produktes wird nach 2 Stunden abgenommen und in einem Rohrreaktor für 20 Minuten auf eine Temperatur von 185 °C erhitzt (Beispiel 1). Danach setzt es sich wie folgt zusammen:

| | |
|---|---|
| Festkörpergehalt | 35 Gew.-% |
| Natriumchloridgehalt | 5,5 Gew.-% |
| Cocosfettsäureaminoamidgehalt | 1,9 Gew.-% |

Der Gehalt an Natriummono- und -dichloracetat ist der Tabelle zu entnehmen.

Der Rohrreaktor besteht aus einem Rohr mit 4,5 mm Innendurchmesser und einer Länge von 3 m. Das Rohr ist mit einem Rohr von 10 mm Innendurchmesser umgeben, mit dessen Hilfe auf einer Länge von 2 m das Produkt mittels Thermalölheizung erhitzt und auf einer Länge von 1 m mit Wasser gekühlt werden kann. Für einen geregelten Durchfluß zur Kontrolle der Verweilzeit sorgt eine Kolbenpumpe mit konstanter Förderleistung, ein Druckhalteventil am Reaktorausgang sorgt dafür, daß Temperaturen von > 100 °C ohne Sieden erreicht werden können.

Ein anderer Teil des Produktes wird nach 8 Stunden Reaktionszeit durch den Rohrreaktor geleitet, wobei eine Temperatur von 185 °C und eine Verweilzeit bei dieser Temperatur von 5 Minuten eingehalten wird (Beispiel 2).

Weitere Teile des Produktes werden nach 8 Stunden Reaktionszeit und Abkühlung durch Zuwaage von Natriummono- und -dichloracetat auf die in der Tabelle angegebenen Konzentrationen an Mono- (MCA) und Dichloressigsäure (DCA) eingestellt. Der pH-Wert der Lösungen ist bekannt und ist ebenfalls in der Tabelle angegeben (Beipiele 3 bis 7).

Die Produkte der Beispiele 2 bis 7 setzen sich wie folgt zusammen:

| | |
|---|---|
| Festkörpergehalt | 35 Gew.-% |
| Natriumchloridgehalt | 5,6 Gew.-% |
| Cocosfettsäureaminoamidgehalt | < 0,3 Gew.-% |

Der Gehalt an Natriummono- und -dichloracetat ist der Tabelle zu entnehmen.

Mit Hilfe der ¹H-NMR-Spektroskopie wird geprüft, ob die Produkte intakt geblieben sind oder sich gegebenenfalls partiell zersetzt haben.

Das nicht erfindungsgemäße Vergleichsbeispiel 8 zeigt, daß sich das Betain nach 30 Minuten/180 °C zersetzt hat.

**Tabelle**

| Beispiel Nr. | Ausgangswert | | | | . | Endwert | | |
|---|---|---|---|---|---|---|---|---|
| | MCA ppm | DCA ppm | pH | Temperatur °C | Zeit Min | MCA ppm | DCA ppm | NMR Zers.* |
| 1 | 1200 | 52 | 8 | 185 | 20 | <10 | <10 | nein |
| 2 | <10 | 35 | 8 | 185 | 5 | <10 | <10 | nein |
| 3 | 1000 | 100 | 8 | 200 | 5 | <10 | <10 | nein |
| 4 | 1000 | 100 | 8 | 200 | 15 | <10 | <10 | nein |
| 5 | 1000 | 20 | 9 | 220 | 1 | <10 | <10 | nein |
| 6 | 5000 | 20 | 7 | 240 | 1 | <10 | <10 | nein |
| 7 | 1000 | 20 | 8 | 240 | 1 | <10 | <10 | nein |
| 8 | 5000 | 100 | 7 | 180 | 30 | nb** | nb** | ja |

| | | | | | | | | |
|---|---|---|---|---|---|---|---|---|
| * = Zersetzung (ja/nein) | | | | | | | | |
| **= nicht bestimmt | | | | | | | | |

## Patentansprüche

1. Verfahren zur Herstellung von Betainen der allgemeinen Formel 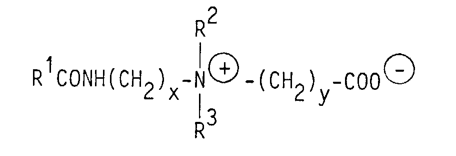 wobei
R¹CO ein von einer Fettsäure oder einem Fettsäuregemisch mit 6 bis 18 Kohlenstoffatomen abgeleiteter Acylrest ist,
R² und R³ gleich oder verschieden sind und Alkylreste mit 1 bis 4 Kohlenstoffatomen bedeuten,
x = 2 oder 3 und
y = 1, 2 oder 3 ist,
durch Quaternierung der Fettsäureamiddialkylamine der allgemeinen Formel 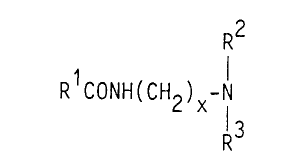 mit ω-Halogenalkylcarbonsäuren der Formel X-(CH₂)_{y}COOH, wobei X ein Halogenrest ist, oder deren Salzen in wäßriger Lösung, dadurch gekennzeichnet, daß man in einer ersten Stufe bei 80 bis 100 °C das Fettsäureamiddialkylamin partiell oder vollständig quaterniert und die Reaktion in einer zweiten Stufe oberhalb einer Temperatur von 180 °C mit einer Reaktionsdauer von 1 bis 25 Minuten durchführt, bis mittels Kapillarelektrophorese kein organisch gebundenes Chlor mehr nachweisbar ist.

2. Verfahren nach Anspruch 1, dadurch gekennzeichnet, daß an die zweite Stufe bei einer Temperatur von 185 bis 250 °C durchführt.

3. Verfahren nach Anspruch 1, dadurch gekennzeichnet, daß man die zweite Stufe in einer Zeit vcn 1 bis 25 Minuten, insbesondere 1 bis 15 Minuten, durchführt.

## Claims

1. Process for the preparation of betaines of the general formula 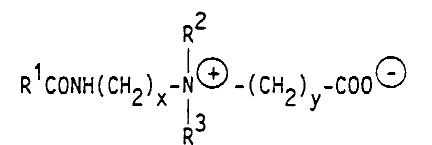 in which
R¹CO is an acyl radical derived from a fatty acid or a fatty acid mixture having from 6 to 18 carbon atoms,
R² and R³ are identical or different and are alkyl radicals having from 1 to 4 carbon atoms,
x = 2 or 3 and
y = 1, 2 or 3,
by quaternization of fatty acid amide dialkylamines of the general formula 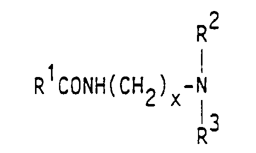 with ω-haloalkylcarboxylic acids of the formula X-(CH₂)_{y}COOH, in which X is a halogen radical, or salts thereof in aqueous solution, characterized in that, in a first stage, the fatty acid amide dialkylamine is partially or completely quaternized at from 80 to 100°C, and the reaction in a second stage is carried out above a temperature of 180°C for a period of from 1 to 25 minutes until organically bonded chlorine is no longer detectable using capillary electrophoresis.

2. Process according to Claim 1, characterized in that the second stage is carried out at a temperature of from 185 to 250°C.

3. Process according to Claim 1, characterized in that the second stage is carried out for a period of from 1 to 25 minutes, in particular from 1 to 15 minutes.

## Revendications

1. Procédé pour la fabrication de bétaïnes de formule générale 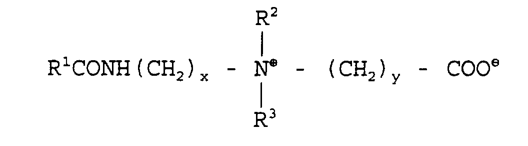 dans laquelle
R¹CO est un radical acyle dérivé d'un acide gras ou d'un mélange d'acides gras comportant de 6 à 18 atomes de carbone,
R² et R³ sont identiques ou différents et représentent des radicaux alkyle comportant de 1 à 4 atomes de carbone,
x = 2 ou 3 et
y = 1, 2 ou 3,
par quaternisation des amidialkylamines d'acides gras de formule générale 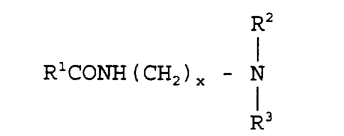 avec des acides ω-halogéno-alkylcarboxyliques de formule X-(CH₂)_{y}COOH, dans laquelle X est un radical halogène, ou ses sels en solution aqueuse, caractérisé en ce que, dans une première étape, de 80 à 100°C, on quaternise partiellement ou totalement l'amidialkylamine d'acide gras, et en ce que l'on effectue dans une seconde étape la réaction à une température supérieure à 180°C, avec une durée de réaction de 1 à 25 minutes, jusqu'à ce que plus aucun chlore lié organiquement ne puisse être mis en évidence par électrophorèse capillaire.

2. Procédé selon la revendication 1, caractérisé en ce que l'on exécute la seconde étape à une température de 185 à 250°C.

3. Procédé selon la revendication 1, caractérisé en ce que l'on exécute la seconde étape en une durée de 1 à 25 minutes, en particulier de 1 à 15 minutes.
